Europäisches Patentamt

European Patent Office

Office européen des brevets

(19)

(11) Publication number: **0 260 762 B1**

(12) **EUROPEAN PATENT SPECIFICATION**

(45) Date of publication of patent specification: **12.01.94**    (51) Int. Cl.5: **C12N 15/80**, C12N 15/90, C12N 15/31, C12N 1/15

(21) Application number: **87201761.1**

(22) Date of filing: **15.09.87**

(54) **Penicillium transformants and methods for their production.**

(30) Priority: **16.09.86 US 907782**

(43) Date of publication of application:
**23.03.88 Bulletin 88/12**

(45) Publication of the grant of the patent:
**12.01.94 Bulletin 94/02**

(84) Designated Contracting States:
**AT BE CH DE ES FR GB GR IT LI LU NL SE**

(56) References cited:
**EP-A- 0 215 539**
**EP-A- 0 225 078**
**EP-A- 0 235 951**
**WO-A-87/05933**

**GENE, vol. 51. no. 1, 1987, pages 97-102, Elsevier Science Publishers B.V.,**

**Amsterdam, NL: F. SANCHEZ et al.: "Transformation in penicillium chrysogenum"**

**BIOTECHNOLOGY, vol. 5, no. 5, May 1987, pages 494-497, New York, US; J.M.CANTORAL et al.: "High-frequency transformation of penicillium chrysogenum"**

(73) Proprietor: **GIST-BROCADES N.V.**
**Wateringseweg 1**
**NL-2611 XT Delft(NL)**

(72) Inventor: **Martin, Juan Francisco**
**Avda. de la Facultad 13-4A**
**E-24004 Leon(ES)**
Inventor: **Alvarez, Emilio**
**Avda. Reina Mercedes 53-6**
**E-41012 Sevilla(ES)**
Inventor: **Barredo, Jose Luis**
**Trespaderne**
**Burgos(ES)**
Inventor: **Cantoral, Jesus Manuel**
**Vega de Monasterio**
**Leon(ES)**
Inventor: **Garcia, Bruno Diez**
**c/Easo 25-4d**
**San Sebastian (Guipuzcoa)(ES)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

APPLIED MICROBIOL BIOTECHNOL, vol. 26, 1987, pages 237-241; U. STAHL et al.."Transformation of penicillium chrysogenum by a vector containing amitochondrial origin of replication"

JOURNAL OF CELLULAR BIOCHEMISTRY SUPPLEMENT (US), vol. 0, no. 9, part C, 1985,page 174; P. VAN SOLINGEN: "Transformation of penicillium chrysogenum"

(74) Representative: **Huygens, Arthur Victor, Dr. et al**
**c/o Gist-Brocades N.V.**
**Patents & Trademarks Department**
**Wateringseweg 1**
**PO Box 1**
**NL-2600 MA Delft (NL)**

**Description**

FIELD OF THE INVENTION

Transformed Penicillium strains are provided for production of novel products. Structural genes are introduced into Penicillium under conditions where the gene is preferably stably integrated into the host genome. The structural gene is expressed providing strains having phenotypic properties of interest.

BACKGROUND OF THE INVENTION

Fungi have found wide use for the preparation of highly functionalized organic compounds. Of particular importance are a broad range of compounds that find use as antibiotics. Processes have been developed for improving fungal strains and for enhancing yields of the desired products in fermentations. Little is known about the regulatory mechanisms that control gene expression during biosynthesis of antibiotics, such as penicillin production by Penicillium chrysogenum (Demain, Antibiotics containing the beta-lactam structure I (Demain and Salomons, eds.) pp. 189-228, Springer-Verlag, 1983; Martin and Liras, Biosynthesis of beta-lactam antibiotics: design and construction of over-producing strains (Trends in Biotechnology 3 - (1985) 39-44). In order to investigate the metabolic mechanisms, it will be necessary in many cases to develop systems where pathways may be identified and manipulated. In this way, the filamentous fungal organisms may be modified to provide more efficient production of indigenous or novel products.

BRIEF DESCRIPTION OF THE RELEVANT LITERATURE

Transformation of Aspergillus nidulans is reported using plasmids carrying a N. crassa fragment comprising the pyr4 gene (Buxton and Redford, Mol. Gen. Genet. 190 (1983) 403-405; Ballance and Turner, Gene 36 (1985) 321-331) and the homologous trpC gene (Yelton et al., Proc. Natl. Acad. Sci. USA 81 (1974) 1470-1474). An A. niger mutant was transformed to prototrophy with the argB gene of A. nidulans (Burton et al., Gene 37 (1985) 207-214. A. nidulans was transformed using a cloned acetamidase gene (Tilburn et al., Gene 26 (1983) 205-221) or the acuD gene (Ballance and Turner, Mol. Gen. Genet. 202 (1986) 271-275) on pBR322. Yeast ura3 mutants can be detected by resistance to 5-fluoroorotic acid (Boeke et al., Mol. Gen. Genet. 197 (1984) 345-346.

Furthermore a transformation of Penicillium chrysogenum has been described in the non-prepublished application EP-A-0235951, using the homologous trpC gene as a marker. The non-prepublished applications EP-A-0240250 and 0215539 disclose the transformation of Penicillium chrysogenum to heterologous drug resistance, viz. to sulphonamide, methotrexate, trimethoprim and G418, respectfully.

SUMMARY OF THE INVENTION

Efficient transformation methods of Penicillium and the resulting transformants are provided. The foreign DNA is preferably stably integrated into the host with stable expression of the structural gene(s) which is introduced. Particularly, complementation of auxotrophy is employed for selection.

DESCRIPTION OF THE SPECIFIC EMBODIMENTS

Efficient transformation of Penicillium is provided to produce transformants having one or more structural genes capable of expression, particularly integrated into the host genome (integrants). DNA constructs are prepared which allow for selection of transformed host cells. Conditions are employed for transformation which result in a high frequency of transformation, so as to ensure selection and isolation of transformed hosts expressing the structural gene(s) of interest. The resulting transformants provide for stable maintenance and expression of the integrated DNA.

For transformation of Penicillium, constructs are employed including at least one marker for selection of transformed cells and, preferably, for enhancing maintenance of the integrated DNA, a transforming enhancing sequence from a filamentous fungus (Ballance and Turner, 1985). Desirably, the marker allows for efficient selection of the transformed host, which selection can be as a result of complementation of an auxotrophic host, resistance to a biocide, e.g. an antibiotic, or the like. The structural gene providing the marker for selection may be native to the wild-type Penicillium host or a heterologous structural gene which is functional in the host. For example, structural genes coding for an enzyme in a metabolic pathway may be derived from Penicillium or from other filamentous fungi, e.g. Aspergillus, Neurospora, Podospora, or yeasts, where the structural gene is functional in the Penicillium host and complements the auxotrophy to prototrophy for the genetic capability.

The complementing structural gene may be derived from a metabolic pathway, such as the synthesis of purines or pyrimidines (nucleosides) or amino acids. Of particular interest are structural genes encoding enzymes in the pyrimidine pathway, e.g. the gene encoding the enzyme orotidine-5'-phosphate decarboxylase (pyrG or pyr4). Other genes of interest are amino acid biosynthesis genes, e.g. ornithine carbamoyl transferase (argB) and arginine succinate lyase (ARG4).

Instead of complementation, biocide resistant genes may be employed. These include genes providing resistance to antibiotics, such as hygromycin, kanamycin, oligomycin, and the like.

For enhanced maintenance, the construct may also include ans like sequences (which may be identified in fungi by its ability to substantially enhance transformation efficiency and under some conditions replicate autonomously, cf. ars in Saccharomyces; Stinchcomb et al., Proc. Natl. Acad. Sci. USA 77 (1980) 4559-4563).

Besides the marker and the optional ans sequence, the construct may include one or more stuctural genes of interest. These genes may be endogenous (homologous) or exogenous (heterologous) genes, particularly genes encoding an enzyme involved with a metabolite of interest, e.g. penicillin or cephalosporin, more particularly a structural gene encoding an enzyme involved with a rate limiting step of the biosynthetic pathway, e.g. tripeptide synthetase, isopenicillin N synthetase (cyclase), transacylase, epimerase, expandase and hydroxylase. Such genes are usually generated by beta-lactam producing microorganisms, such as Penicillium, Cephalosporium, Streptomyces, and the like. Also, genes which encode enzymes, structural proteins, receptors or the like may be of interest. Instead of endogenous genes, exogenous genes may be employed, particularly from other filamentous fungi, which encode enzymes which have the same or different function from an endogenous enzyme. These genes include the glucoamylase, alpha-amylase, cellulase, protease, lipase, ligninase and rennet genes, etc.

Exogenous or heterologous genes may be introduced to provide novel functions for the Penicillium host. These functions may involve the transformation of an endogenous metabolite, the production of a novel non-proteinaceous product or the production of a novel proteinaceous product. The Penicillium may be efficiently fermented to produce a wide variety of products of interest.

Proteins of interest include mammalian proteins, e.g. chymosin, and human proteins, such as lymphokines, blood factors, e.g. clotting factors, interferons, growth factors, hormones, etc. By being a eukaryote, Penicillium offers an opportunity to produce protein products which are processed analogously to vertebrate processing.

The structural genes will be under the transcriptional and translational control of initiation and termination regions which are functional in Penicillium, which regions may be endogenous or exogenous, particularly filamentous fungal regions. The transcriptional initiation may be constitutive or regulated, usually being a strong initiation region. Where the structural gene has a naturally occurring regulatory region which is functional in Penicillium and has the appropriate order of activity, the region may be employed with the structural gene. Alternatively, a regulatory region other than the natural region may be joined to the structural gene. Regulatory regions of interest include the initiation regions of the pyr4, pyrG, acuD, npe, trpC, argB, or strong promoters derived from the glycolytic pathway (e.g. PGK, GAPDH, TPI), or the control regions of highly expressed enzymes, such as glucoamylase and cellulase, or those involved in the penicillin biosynthetic pathway, e.g. isopenicillin N synthetase (IPNS). These genes have been or may be readily isolated and their regulatory regions defined and joined in appropriate order to the structural gene.

The pyr4 gene is highly expressed in P. chrysogenum as a result of high copy number and/or a high frequency of transcription. The pyr4 insert in the subject constructs provides the opportunity for efficient expression of structural genes under the regulatory control of the pyr4 transcriptional initiation region. The gene of interest may be inserted by appropriate manipulation of the pyr4 gene, employing available restriction sites or introducing restriction sites by mutagenesis, by resection, or the like, where the gene of interest may be ligated to the 5'-non-coding regulatory region of the pyr4 gene or inserted into the pyr4 gene in reading phase to provide a fused amino acid sequence resulting from the presence of one or more codons at the 5'-terminus of the coding sequence.

As vectors, the plasmids pDJB1 and pDJB2 find use or may serve as a pyr4 gene and the ans sequence. These plasmids may be obtained after amplification overnight in E. coli in medium containing 300 $\mu$l/ml spectinomycin, followed by cesium chloride-ethidium bromide ultracentrifugation employing standard methods (Maniatis et al., (1982) Molecular Cloning, A Laboratory Manual. Cold Spring Harbor Laboratory, Cold Spring Harbor, NY).

In the preparation of the construct, the various fragments may be cloned in E. coli on an appropriate cloning vector, e.g. pBR322, pACYC184, pUC, etc. The fragments may be joined and/or modified employing convenient restriction sites, linkers, adapters, primer repair, in vitro mutagenesis, resection, or the like. The fragments may be joined by ligation, cloned, analysed by restriction enzyme analysis or sequencing and then used to combine with other fragments, the process being repeated until the construct is complete. Since stable extrachromosomal maintenance is not required, the cloning vector may be employed as part of the transforming DNA, either linearized or circular. Alternatively, the construct may be excised from the vector for transformation. While the DNA comprising the construct may be more than one piece, where the structural genes and regulatory regions will be intact, usually the construct will be a single piece of DNA.

For efficient transformation, selected conditions will be employed. The Penicillium may be wild-type or laboratory strains, prototrophic or auxotrophic in one or more metabolic pathways, or mutants blocked in penicillin biosynthesis. The Penicillium may be employed as protoplasts substantially free of intact mycelia. The intact mycelia may be removed after degradation of the cell wall. The conditions for cell wall removal and removal of intact mycelia are conventional. Lysis is carried out for from 2 to 6 h at ambient conditions, employing about 0.1 to 1 mg/ml of enzyme at a pH in the range of about 5 to 6 in an isotonic medium, e.g. 0.7 M KCl. The mycelia are present at about 25 to 150 mg wet weight/ml. The protoplasts may be obtained as a filtrate in an isotonic medium containing from about 25 to 100 mM $Ca^{++}$ ions.

The transformation is performed with about $10^7$-$10^9$ protoplasts/ml using polyethylene glycol as the fusogen with about 1-10 $\mu$g DNA in a Tris EDTA (TE) buffer containing a low concentration of calcium ions and at a pH of about 5.5 to 7.5, particularly 5.8. After a short period at low temperatures, e.g. 0-5°C, 15 to 45 min, the solution is diluted about 5-20 fold with a different buffered medium which is about 20-60%, preferably 50%, polyethylene glycol and contains 25-75 mM, preferably 50 mM $CaCl_2$ and about 5 to 25 mM MOPS (N-morpholino propanesulfonic acid) and incubated for 10 to 30 min. at ambient temperatures, e.g. 20-30°C. The polyethylene glycol may be PEG 1000-8000, preferably 8000.

After incubation, the medium is further diluted and the protoplasts plated onto regeneration medium, e.g. Czapek-sucrose minimal medium (J.F. Peberdy, Fungal protoplasts: isolation, reversion and fusion, Ann. Rev. Micr. 33 (1979) 21-39).

The regeneration will provide for selection of transformants. The resulting colonies may be resuspended in an appropriate nutrient medium, recloned, and analysed for expression of the desired product.

The resulting transformants may result from homologous or random integration and may have one or more constructs integrated into the genome. Where homologous recombination is desired, native DNA may be employed as part of the construct, where the native DNA may result in single or double cross-over recombination. The native DNA may be chosen to result in inactivation of a wild-type gene, to substitute for the native DNA without gene inactivation, or to integrate into an innocuous region.

The transformants may be of various Penicillium strains, the preferred strain being Penicillium chrysogenum.

The transformed strains may be used in fermentors in fermentation processes for the production of products which may be retained in the cytoplasm or secreted into the medium. The secretion will depend upon the recognition by the host of an appropriate signal sequence.

Conditions for fermentation with Penicillium are well known. See, for example, G.J.M. Hersbach, C.P. v.d. Beek and P.W.M. van Dijck, The Penicillins: Properties, Biosynthesis and Fermentation, in Biotechnology of Industrial Antibiotics, E. van Damme (ed.), Marcel Dekker 1984.

The following examples are offered by way of illustration and not by way of limitation.

Example 1

Transformation of a Penicillium chrysogenum pyrG auxotroph using the Neurospora crassa pyr4 gene

PyrG auxotrophs of P. chrysogenum Wis 54-1255 (pyrG, $Aza^R$) and of mutants derived from it (pyrG, $Aza^R$, npe) blocked in penicillin production were used as recipient strains. PyrG auxotrophs were readily isolated among mutants resistant to 5-fluoro-orotic acid (Boeke et al., Mol. Gen. Genet. 197 (1984) 345-346). Conidia of the different strains were inoculated in minimal medium (100 ml) supplemented with 0.2%, yeast extract (Anne and Peberdy, Induced fusion of fungal protoplasts following treatment with polyethylene glycol., J. Gen. Microbiol. 92 (1976) 413-417) at a final concentration of $10^7$ conidia/ml. Flasks were incubated in an orbital shaker (250 rpm) at 28°C for 20 to 48 h (wild type or auxotrophs respectively). The mycelium was recovered by filtration in a nylon filter (Nytal, 30 $\mu$m-pore) washed with NaCl (9 g/l) and dried by pressing between two filter papers. To obtain protoplasts the mycelium was resuspended (100 mg of wet weight/ml) in lytic buffer (50 mM phosphate buffer pH 5.8, containing 0.7 M KCl). An identical volume of Novozym 234 (Novo Industries Ltd, Copenhagen, Denmark) (1 mg/ml in lytic buffer) was added.

5

The lytic mixture was incubated with gentle shaking for 3 h at 25°C following microscopically the release of protoplasts. The residual mycelium was removed by filtering the protoplasts through a 30 μm-pore sterile nylon filter. The protoplasts were washed 3 times with 0.7 M KCl by centrifugation (400 g, 3 min) collected and resuspended in 100 ml of KCM solution (0.7 M KCl, 50 mM CaCl$_2$, 10 mM MOPS, pH 5.8).

Protoplasts were counted microscopically with a counting chamber and adjusted with KCM solution to a final concentration of $10^8$ protoplasts/ml. In the standard procedure for transformation, 50 μl of protoplast suspension were mixed with 1 to 10 μg of DNA in TE buffer and with 12 μl of PCM (50% polyethyleneglycol 8000, 50 mM CaCl$_2$; 10 mM MOPS pH 5.8). Each reaction was mixed thoroughly and kept at 4°C for 30 min. 500 μl of PCM were added later and incubated at 25°C for 20 min. Finally, 1 ml of solution was added and after any further dilutions, the protoplasts were plated in an overlay of Czapek-sucrose minimal medium (0.7 M KCl). A modified procedure in which 250 (or 500) μl of protoplast suspension and the corresponding amounts of the other components was used in some experiments.

Transfer of DNA to nitrocellulose, nick-translation, restriction enzyme digestion and ligation were carried out by standard procedures (Manitatis et al., 1982).

The Penicillium chrysogenum strain Wis 54-1255 (pyrG, Aza[R]) was deposited on September 16, 1986 with the Commonwealth Mycological Institute at Kew, United Kingdom, and given deposit number CMI 309919.

The results of different transformation experiments in which the effect of several variables were studied are shown in Tables 1 and 2.

## Table 1

## Effect of DMSO, number of protoplasts and DNA concentration on transformation of P. chrysogenum Wis 54-1255 Strain (pyrG, Aza[R])

| Trans- formation assay | Proto- plasts (μg) | DMSO (μl) | Plasmid DNA pDJB2 | PCM Mixture (μl) | PCM Dilution (ml) | Transformants μg of DNA |
|---|---|---|---|---|---|---|
| 1 | 500 | 5 | 8 | 50 | 2 | $1.2 \times 10^3$ |
| 2 | 500 | 0 | 8 | 50 | 2 | $1.3 \times 10^3$ |
| 3 | 500 | 5 | 12 | 50 | 2 | $2.8 \times 10^3$ |
| 4 | 500 | 5 | 20 | 50 | 2 | $2.2 \times 10^3$ |
| 5 | 250 | 2 | 28 | 25 | 1 | $3.0 \times 10^3$ |
| 6 | 250 | 2 | 14 | 25 | 1 | $3.0 \times 10^3$ |
| 7 control | 500 | 5 | none | 50 | 2 | 0 |

6

Table 2

Effect of the concentration of polyethyleneglycol and the
increasing concentrations of ~DNA on~ transformation of
P. chrysogenum Wis 54-1255 Strain (pyrG, AzaR)

| Trans-formation assay | Proto-plasts (μg) | Plasmid DNA (μg) | PCM Mixture (μl) | PCM Dilution (ml) | KCM Solution (ml) | Transformants/ μg of DNA |
|---|---|---|---|---|---|---|
| 1 | 50 | 0.4 | 12(50% PEG) | 0.5(50%PEG) | 1 | $0.3 \times 10^3$ |
| 2 | 50 | 2.0 | 12 " | 0.5 " | 1 | $1.9 \times 10^3$ |
| 3 | 50 | 4.0 | 12 " | 0.5 " | 1 | $0.9 \times 10^3$ |
| 4 | 50 | 0.4 | 12(25%PEG) | 0.5(25%PEG) | 1 | $0.11 \times 10^3$ |
| 5 | 50 | 2.0 | 12 " | 0.5 " | 1 | $0.45 \times 10^3$ |
| 6 | 50 | 4.0 | 12 " | 0.5 " | 1 | $0.15 \times 10^3$ |
| 7 control | 50 | none | 12 " | 0.5 " | 1 | 0 |

The reversion frequency of the different uracil auxotrophs (pyrG) used as host strains was in all cases less than one in $2 \times 10^8$, i.e. no revertants were obtained in a plate seeded with $2 \times 10^8$ protoplasts.

The highest efficiency of transformation was obtained using a plasmid DNA concentration in the range of 12-28 μg of DNA and about $2.5 \times 10^7$ protoplasts per transformation reaction. The addition of DMSO did not affect the efficiency of transformation.

The frequency of transformation that was obtained is relatively high in comparison with the frequencies obtained in other fungi (Johnstone, Transformation of Aspergillus nidulans Microbiol. Sci. 2, (1985) 307-311). In several fungi, transformation frequencies are in the range of 10 to 20 transformants/μg of DNA (Ballance et al., 1983; Tilburn et al., 1984; Wernars et al., Gene amplification in Aspergillus nidulans by transformation with vectors containing amdS gene, Current Genetics 9, (1985) 361-368). The efficiency of transformation in the subject examples is similar to the high frequency reported recently by Ballance and Turner (1985) in A. nidulans.

The location of the plasmid DNA after transformation was studied by hybridization using [32]P-labelled pBR322 as a probe, since this plasmid forms part of pDJB1 and pDJB2. pBR322 was used as a probe since it does not contain fungal DNA sequences that might be homologous to the chromosomal DNA of P. chrysogenum. No hybridization with total DNA of untransformed P. chrysogenum was obtained. However clear hybridization bands were obtained with total DNA of several transformant strains digested with either PvuII, EcoRI or BclI. The DNA fragments that give positive hybridization were not identical to the size of pDJB2 integrated into the chromosome. An additional proof of the absence of pDJB2 as free plasmids in the transformants is the lack of detection by hybridization of free plasmid in undigested total DNA of transformed strains.

In S. cerevisiae, Hinnen et al., (Transformation of yeast., Proc. Natl. Acad. Sci. USA 75 (1978) 1929-1933) described three types of integration: types I and III involved homologous recombination (by single and double crossovers, respectively) between the transforming DNA and the equivalent host gene; type II integration occurred at other sites in the genome. In Aspergillus, in some cases, integration occurs predominantly at the site of homology. For example, Tilburn et al. (1984), suggested that plasmids carrying the amdS structural gene plus part of the Aspergillus ribosomal DNA repeat were integrated into the ribosomal DNA.

However, integration can also occur at non-homologous sites. In the case of the pyr4 gene of N. crassa there is no significant homology with the Aspergillus pyrG locus and integration occurs elsewhere (Ballance et al., Biochem. Biophys. Res. Comm. 112 (1983). Yelton et al., (1984) and Ballance and Turner (1985) also reported type II integration in A. nidulans using a trpC marker. The pyr4 gene or N. crassa, which codes for orotidine-5'-phosphate decarboxylase, is capable of transforming an A. nidulans pyrG mutant by chromosomal integration despite low homology between the transforming DNA and the recipient genome (Ballance and Turner, 1985, supra). The situation is probably similar with P. chrysogenum although the exact degree of homology between the Neurospora and Penicillium pyr4 genes is not known. Since a heterologous ans sequence from A. nidulans DNA is used, the possibility of integration in a nonhomologous site exists as long as the integration event does not disrupt any gene essential for viability on the selective regeneration medium.

Two kinds of transformant colonies have been found. The first type are large colonies that grow normally in non-selective medium. There are also small colonies that apparently experience transient expression of the pyr4 gene, but are not normally able to develop into mature transformants and to replicate after subculturing in selective medium. Similar findings were reported in the transformants of A. nidulans with the acetamidase gene (Tilburn et al., 1983; Wernars et al., Current Genetics 9 (1985) 361-368).

There is a clear effect of the ansl sequence (present in pDJB2) in transformation of P. chrysogenum using the N. crassa pyr4 gene. The number of stable transformants obtained using pDJB2 was 15-fold higher than the number obtained using pDJB1 lacking the ansl sequence but otherwise identical to pDJB2. The low number of abortive transformants with the ans containing vector is suggestive of early stable integration. Similar results have been reported by Ballance and Turner, 1985. These authors suggested that pDJB2 (as different from pDJB1) might undergo several rounds of replication upon entry into the protoplasts prior to integration. This would also explain enhanced transformation and the reduction in the number of abortive transformants observed in P. chrysogenum using pDJB2.

The expression of the pyr4 gene in P. chrysogenum transformants was studied by assaying for orotidine-5′-monophosphate (OMP) activity by detecting uracil-5′-monophosphate (UMP). A low OMP decarboxylase activity was found in wild-type cultures and almost no activity in a P. chrysogenum pyrG mutant. The results support a high level of expression of the pyr4 gene in Penicillium, see Table 3.

## Table 3

### OMP-decarboxylase activity of the wild type and a transformant strain of P. chrysogenum using the pyr$^4$ gene

| Strains | OMP-decarboxylase (specific activity) mU/mg of protein | % of wild type |
|---|---|---|
| P. chrysogenum Wis 54-1255 (wild type) | 62 | 100 |
| P. chrysogenum Wis 54-1255 pyrG | 0 | 0 |
| P. chrysogenum Wis 54-1255 T$_2$ (pDJB2) | 292 | 471 |

One unit of the enzyme (U) is the activity forming 1 μmole of UMP in 1 h, at 30°C in 50 mM Tris buffer pH 8.0.

OMP-decarboxylase activity was assayed using a radiometric assay to determine the conversion of [6-$^{14}$C] OMP into [6-$^{14}$C] UMP. Both nucleotides were separated by TLC using PEI-cellulose plates developed in 0.3M lithium chloride.

Example 2

Transformation of a P. chrysogenum pyrG auxotroph using the homologous P. chrysogenum pyrG gene

In order to isolate the pyrG gene of P. chrysogenum a genomic library of wild-type P. chrysogenum DNA was constructed in the SalI site of the bacteriophage lambda vector pEMBL 3 (Frischauf et al., J. Mol. Biol. 170, 827-842 (1983) by standard methods (Maniatis et al. (1982)).

This library was screened by plaque hybridization in 45% formamide, using the heterologous N. crassa pyr4 gene as a probe. Seven recombinant phages were obtained which gave a positive hybridization signal. These phages had an average insert size of 17 kilobases. Upon digestion with restriction enzyme SalI, all had insert fragments of 3.07 and 2.66 kb in common. The enzyme BamHI generated three fragments common to all phages, with sizes of 3.6, 1.1, and 0.75 kb, respectively.

The fragments carrying the pyrG gene were identified by Southern hybridization, using as a probe the sythetic oligonucleotide mixture 5′d (TTC GAG CAPy CGPy AAG TTC)3′, corresponding to a conserved region in the N. crassa pyr4 and Saccharomyces cerevisiae URA3 genes (Newbury et al., Gene 43 (1986) 51-58). The 3.6 kb BamHI fragment and the 3.07 kb SalI fragment, which is internal to the former, gave a positive signal.

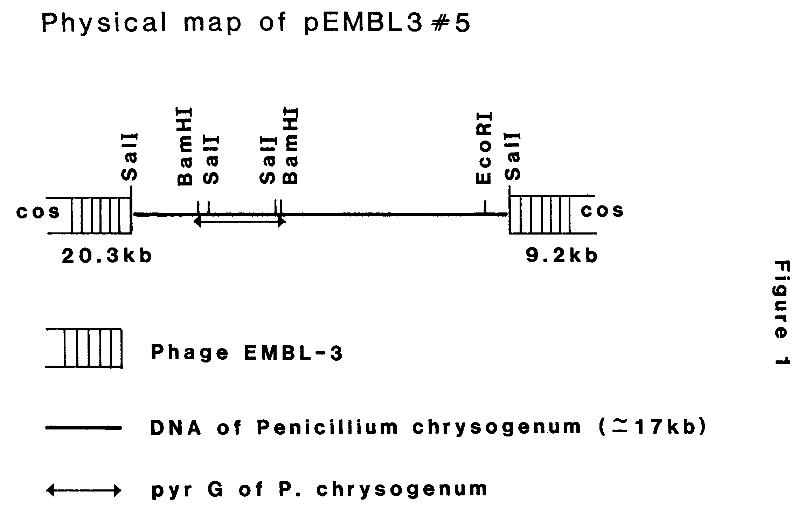

One out of the seven positive recombinants, pEMBL3/5 (= pEMBL3#5, see physical map shown in Fig. 1) was chosen for transformation of pyrG auxotrophic P. chrysogenum Wis-54-1255 recipients as described in Example 1. Prototrophic transformants were obtained at a frequency of 50-100 per microgram of added pEMBL3/5 DNA.

Phage pEMBL3/5 was deposited with the Centraal Bureau voor Schimmelcultures, Baarn, Netherlands, on August 28, 1987 under CBS 399.87.

A 4 kb Sau3A partial fragment contained within the insert of plasmid pEMBL 3/5 was subcloned in the BamHI site of plasmid pUCl3. The resulting construct (pUCl3::pyrG) was successfully used to transform the Penicillium chrysogenum pyrG recipient strain with high frequency. E. coli strain DHI containing this plasmid was deposited with CBS on September 14, 1987 under CBS 426.87.

Example 3

Transformation of P. chrysogenum arginine auxotrophs using the Saccharomyces cerevisiae ARG4 gene

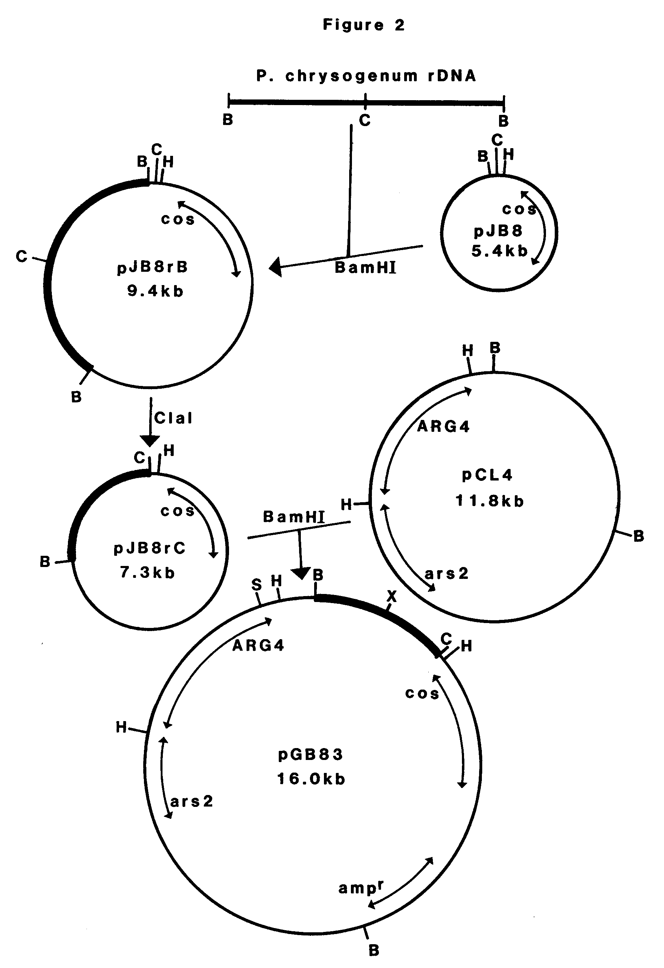

The donor plasmid, pGB83 that was used in this Example was constructed from parts of the cosmid vector pJB8 (Ish-Horowicz and Burke, Nucl. Ac. Res. 9 (1981) 2989-2998), the E. coli/ S. cerevisiae hybrid plasmid pCL4 (Hsiao and Carbon, Gene 15 (1981) 157-166), which contains the yeast ARG4 (argininosuccinate lyase) gene, and 2 kilobases of the P. chrysogenum ribosomal DNA repeat, in order to promote recombination between the donor plasmid and the host genome. The ribosomal DNA-containing cosmid pJB8rB was identified in a Penicillium genomic library using [32]P-labelled Penicillium ribosomal RNA as a hybridization probe. Details of the construction are outlined in Figure 2, in which the abbreviations B, C, H, S and X denote restrictions sites for the enzymes BamHI, ClaI, HindIII, SalI, and XbaI, respectively.

As recipient strains, seven different arginine-requiring auxotrophs of P. chrysogenum were used. Transformation was carried out essentially as described in Example 2. With two mutants, HDM2 and HDM15, transformants were obtained. Transformation frequency was usually in the order of 1 transformant per microgram of added DNA, although in some experiments, using HDM15 as the recipient, as many as 100 transformants per microgram could be obtained.

The transformant character of the clones obtained was verified by hybridization of total DNA, isolated from the transformants, with the vector plasmid, pJB8.

Example 4

Transformation of a P. chrysogenum argB auxotroph using the Penicillium chrysogenum argB gene

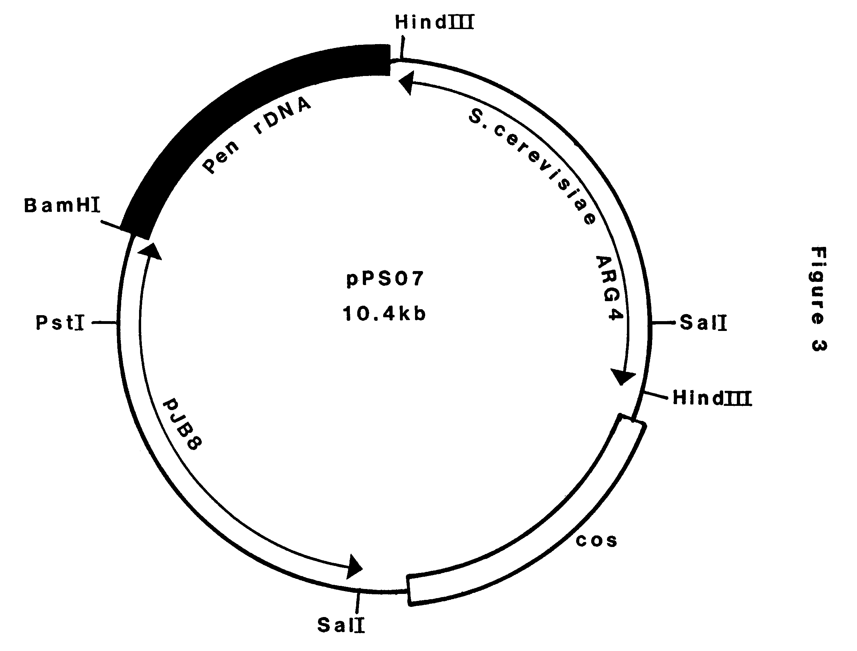

In order to isolate the P. chrysogenum argB (ornithine carbamoyltransferase) gene, a genomic library of wild-type P. chrysogenum was constructed by standard methods (see Example 2) in cosmid vector pPS07 (see Figure 3). This vector is a derivative of the cosmid pJB8, containing the S. cerevisiae ARG4 gene, and 2 kilobases of P. chrysogenum ribosomal DNA (see also Example 3 and the physical map in Figure 1).

The size of inserts of P. chrysogenum DNA in the library ranged from 10 to 30 kilobases. The library was subdivided into 32 pools, each containing 12 clones. Cosmid DNAs were isolated from the pooled clones, digested with Eco RI, and transferred to nitrocellulose filters, using standard procedures (Maniatis et al., 1982). The transfers were screened by hybridization at room temperature in 50% formamide, using the heterologous Aspergillus nidulans argB gene (Berse et al. Gene 25 (1983) 109-117) as a probe.

In one of the pools, a hybridizing EcoRI fragment was present with the same electrophoretic mobility (1.7 kb) as the hybridization signal observed in EcoRI-digested P. chrysogenum chromosomal DNA. The individual clone which gave the positive signal was isolated from the pool and designated pRV130. The 1.7 kb EcoRI fragment was isolated from the pRV130 insert and subcloned on plasmid vector pUCl9 (Yanisch-Perron et al., Gene 33 (1985) 103-119) giving plasmid pRV01. Both plasmids were successfully used in transformation experiments to be described below.

argB auxotrophs of P. chrysogenum were used as recipient strains. These may be readily identified among arginine auxotrophs by their ability to grow on citrulline-supplemented minimal medium but not on ornithine-supplemented media.

Transformation was performed following the method described in the previous Examples. In a typical experiment, 10 micrograms of donor DNA was used; the frequency of transformation was about 1 transformant per microgram.

Occurence of transformation was further confirmed by hybridization of transformant DNA with the vector plasmid, e.g. pJB8.

The subject invention provides an effective procedure for the transformation of Penicillium and stable maintenance of the introduced gene(s) of interest to provide strains having desired properties. Conditions are provided which result in a high frequency of transformation, so that clones may be obtained which provide optimum results.

Although the foregoing invention has been described in some detail by way of illustration and examples for purposes of clarity of understanding, it will be obvious that certain changes and modifications may be practices within the scope of the appended claims.

## Claims

1. A transformed Penicillium strain, comprising at least one DNA construct comprising a structural gene which complements an auxotrophic Penicillium to prototrophy, with the exception of trpC.

2. A transformed Penicillium strain according to Claim 1, wherein said structural gene is from Penicillium.

3. A transformed Penicillium strain according to Claim 1, wherein said structural gene is from a source other than Penicillium.

4. A transformed Penicillium strain according to any of Claims 1-3, wherein said construct comprises a filamentous fungal gene for orotidine-5'-phosphate decarboxylase.

5. A transformed Penicillium strain according to Claim 1, of the species chrysogenum.

6. A transformed Penicillium strain according to Claim 1, wherein said construct comprises a transcriptional initiation regulatory region selected from the group consisting of pyr4, pyrG, argB and ARG4.

7. A transformed Penicillium strain according to Claim 1, wherein said construct comprises the ans gene.

8. A transformed Penicillium strain according to Claim 7, wherein said construct comprises a filamentous fungal structural gene for a metabolite in the biosynthetic pathway to a purine or pyrimidine or amino acid and said Penicillium strain is complemented by said structural gene to prototrophy.

9. A method for transforming a Penicillium host cell wherein said host cell is an auxotroph, except trpC, which comprises:

combining protoplasts of said host cell with a DNA construct comprising a structural gene which when expressed is capable of providing a prototroph as to at least one metabolic pathway of said host cell, under transforming conditions so as to provide transformed protoplasts wherein said DNA construct is integrated at least in part into the genome of said host cell; and

growing said transformed protoplasts under cell wall regenerating and prototrophy selective conditions to provide Penicillium transformants.

10. A method according to Claim 9, wherein the DNA construct further comprises the ans gene.

11. A method according to Claim 9 or 10, wherein said DNA construct is integrated at least in part into the genome of said host.

12. A method according to any one of Claims 9-11, wherein said auxotrophy is in the biosynthetic pathway to a purine of pyrimidine or an amino acid.

13. A method according to any one of Claims 9-12, wherein said structural gene is orotidine-5'-phosphate decarboxylase, arginine succinate lyase or ornithine carbamoyl transferase.

14. A method according to any one of Claims 9-13, wherein said construct includes a gene of interest capable of expression in said Penicillium to produce a product of interest.

15. A method according to Claim 14, wherein said gene of interest is under the transcriptional initiation regulation of the pyr4, pyrG, argB or ARG4 gene transcriptional initiation region.

16. A method for producing a desired protein in Penicillium which comprises:
    growing a Penicillium strain produced according to any one of the methods of Claims 9-15, wherein said gene is expressed to produce said product of interest.

**Patentansprüche**

1. Transformierter Penicillium-Stamm, dadurch gekennzeichnet, dass er mindestens ein DNA-Konstrukt aufweist, das ein Strukturgen aufweist, das durch Komplementation einen auxotrophen Penicillium-Stamm prototroph macht, mit Ausnahme von trpC.

2. Transformierter Penicillium-Stamm nach Anspruch 1, dadurch gekennzeichnet, dass das genannte Strukturgen aus Penicillium stammt.

3. Transformierter Penicillium-Stamm nach Anspruch 1, dadurch gekennzeichnet, dass das genannte Strukturgen aus einer Quelle stammt, die von Penicillium verschieden ist.

4. Transformierter Penicillium-Stamm nach einem der Ansprüche 1 bis 3, dadurch gekennzeichnet, dass das genannte Konstrukt ein Fadenpilzgen für Orotidin-5'-phosphat-Decarboxylase aufweist.

5. Transformierter Penicillium-Stamm nach Anspruch 1 der Spezies chrysogenum.

6. Transformierter Penicillium-Stamm nach Anspruch 1, dadurch gekennzeichnet, dass das genannte Konstrukt eine regulatorische Region zur Transkriptionsinitiation aufweist, die aus der Gruppe gewählt ist, die aus pyr4, pyrG, argB und ARG4 besteht.

7. Transformierter Penicillium-Stamm nach Anspruch 1, dadurch gekennzeichnet, dass das genannte Konstrukt das ans-Gen aufweist.

8. Transformierter Penicillium-Stamm nach Anspruch 7, dadurch gekennzeichnet, dass das genannte Konstrukt ein Fadenpilz-Strukturgen für einen Metaboliten in der Biosynthese eines Purins oder Pyrimidins oder einer Aminosäure aufweist und der genannte Penicillium-Stamm durch Komplementation durch das genannte Strukturgen prototroph gemacht wird.

9. Verfahren zum Transformieren einer Penicillium-Wirtzelle, wobei die genannte Wirtzelle auxotroph ausser für trpC ist, dadurch gekennzeichnet, dass man
    Protoplasten der genannten Wirtzelle unter transformierenden Bedingungen mit einem DNA-Konstrukt kombiniert, das ein Strukturgen aufweist, durch dessen Expression ein in bezug auf mindestens einen Stoffwechselweg der genannten Wirtzelle prototropher Stamm erzeugt werden kann, um transformierte Protoplasten zu erzeugen, in denen das genannte DNA-Konstrukt mindestens zum Teil in das Genom der genannten Wirtzelle integriert ist; und
    die genannten transformierten Protoplasten unter Bedingungen zur Regeneration der Zellwandung

EP 0 260 762 B1

und zur Selektion prototropher Stämme kultiviert, um Penicillium-Transformanten zur Verfügung zu stellen.

10. Verfahren nach Anspruch 9, dadurch gekennzeichnet, dass das DNA-Konsturukt ferner das ans-Gen aufweist.

11. Verfahren nach Anspruch 9 oder 10, dadurch gekennzeichnet, dass das genannte DNA-Konstrukt mindestens zum Teil in das Genom des genannten Wirts integriert ist.

12. Verfahren nach einem der Ansprüche 9 bis 11, dadurch gekennzeichnet, dass sich die genannte Auxotrophie auf einen Metaboliten in der Biosynthese eines Purins oder Pyrimidins oder einer Aminosäure bezieht.

13. Verfahren nach einem der Ansprüche 9 bis 12, dadurch gekennzeichnet, dass das genannte Strukturgen Orotidin-5'-phosphat-Decarboxylase, Argininsuccinatlyase oder Ornithincarbamoyltransferase ist.

14. Verfahren nach einem der Ansprüche 9 bis 13, dadurch gekennzeichnet, dass das genannte Konstrukt ein interessierendes Gen einschliesst, das zur Expression in dem genannten Penicillium-Stamm befähigt ist, um ein interessierendes Produkt zu erzeugen.

15. Verfahren nach Anspruch 14, dadurch gekennzeichnet, dass die Transkriptionsinitiation des interessierenden Gens durch die Transkriptionsinitiationsregion des pyr4-, pyrG-, argB- oder ARG4-Gens reguliert wird.

16. Verfahren zur Erzeugung eines gewünschten Proteins in Penicillium, dadurch gekennzeichnet, dass man einen Penicillium-Stamm, der gemäss dem Verfahren nach einem der Ansprüche 9 bis 15 erzeugt ist, kultivert, wobei das genannte Gen exprimiert wird, um das genannte interessierende Produkt zu erzeugen.

**Revendications**

1. Souche de Penicillium transformée, comprenant au moins une construction d'ADN constitué d'un gène de structure qui complémente un Penicillium autotrophe en prototrophe, avec l'exception de trpC.

2. Souche Penicillium transformée suivant la revendication 1, où le gène de structure provient de Penicillium.

3. Souche de Penicillium transformée suivant la revendication 1, où le gène de structure provient d'une source autre que du Penicillium.

4. Souche de Penicillium transformée suivant l'une quelconque des revendications 1 à 3, où la construction comprend un gène de champignon filamenteux pour l'orotidine-5'-phosphate décarboxylase.

5. Souche de Penicillium transformée suivant la revendication 1, de l'espèce chrysogenum.

6. Souche de Penicillium transformée suivant la revendication 1, où la construction comprend une région de régulation d'initiation de la transcription choisie parmi le groupe consistant en pyr4, pyrG, argB et ARG4.

7. Souche de Penicillium transformée suivant la revendication 1, où la construction comprend le gène ans.

8. Souche de Penicillium transformée suivant la revendication 7, où la construction comprend un gène de structure de champignon filamenteux pour un métabolite d'un cycle de biosynthèse de purine ou de pyrimidine ou d'un acide aminé et la souche de Penicillium est complémentée par le gène de structure en prototrophe.

9. Procédé de transformation d'une cellule hôte de Penicillium, où la cellule hôte est un auxotrophe, sauf trpC, qui comprend :

la combinaison de protoplastes de la cellule hôte avec une construction d'ADN comprenant un gène de structure qui, quand il est exprimé, est capable de fournir un prototrophe pour ce qui est d'au moins un cycle métabolique de la cellule hôte, dans des conditions de transformation telles qu'on obtient des protoplastes transformés, où la construction d'ADN est intégrée au moins en partie dans le génome de la cellule hôte, et

la croissance des protoplastes transformés dans des conditions sélectives de régénération de la paroi cellulaire et de prototrophie pour fournir des transformants Penicillium.

10. Procédé suivant la revendication 9, où la construction d'ADN comprend de plus le gène ans.

11. Procédé suivant la revendication 9 ou 10, où la construction d'ADN est intégrée au moins en partie dans le génome de l'hôte.

12. Procédé suivant l'une quelconque des revendications 9 à 11, où l'auxotrophie se situe dans le cycle biosynthétique d'une purine ou d'une pyrimidine ou d'un acide aminé.

13. Procédé suivant l'une quelconque des revendications 1 à 12, où le gène de structure est de l'orotidine-5'-phosphate décarboxylase, de l'arginine succinate lyase ou de l'ornithine carbamoyltransférase.

14. Procédé suivant l'une quelconque des revendications 9 à 13, où la construction comprend un gène intéressant capable d'expression dans le Penicillium pour produire un produit intéressant.

15. Procédé suivant la revendication 14, où le gène intéressant est sous régulation d'initiation de transcription de la région d'initiation de transcription du gène pyr4, pyrG argB ou ARG4.

16. Procédé pour la production d'une protéine désirée dans Penicillium qui comprend :
la croissance d'une souche Penicillium produite suivant l'un quelconque des procédés des revendications 9 à 15, où le gène est exprimé pour produire le produit intéressant.

13

# Physical map of pEMBL3#5

cos | Sal| | | BamHI | Sal| | | Sal| | BamHI | | EcoRI | Sal| | cos

**20.3kb**  **9.2kb**

Phage EMBL-3

DNA of Penicillium chrysogenum ($\simeq$17kb)

pyr G of P. chrysogenum

**Figure 1**

EP 0 260 762 B1

## Figure 2

# Figure 3